# EUROPEAN PATENT APPLICATION

(11) **EP 4 088 735 A1**
(43) Date of publication of application: **16.11.2022**
(21) Application number: 20905087.1
(22) Date of filing: 28.08.2020
(51) Int. Cl.: A61K 38/57, A61P 1/16

(54) **APPLICATION OF CST1 IN PREVENTION AND/OR TREATMENT OF LIVER IMMUNE DYSREGULATION DISEASES**

(30) Priority: 25.12.2019 CN 201911358054
(71) Applicant: Center for Excellence in Molecular Cell Science, Chinese Academy of Sciences, Shanghai 200031 (CN)
(72) Inventor: CHENG, Xin, Shanghai 200031 (CN); XU, Yilin, Shanghai 200031 (CN)
(74) Representative: Rondano, Davide
(86) International application number: PCT/CN2020/112234
(87) International publication number: WO 2021/128919

(57) **Abstract**

The present invention provides an application of CST1 in prevention and/or treatment of liver immune dysregulation diseases. Specifically, the present invention provides an application of a CST1 gene, or a protein thereof, or a promoter thereof for preparing a composition or a preparation. The composition or the preparation is used for prevention and/or treatment of liver immune dysregulation diseases.

## Description

### Technical field

The present invention relates to the field of biomedicine, in particular, the present invention relates to the application of CST1 in preventing and/or treating liver immune disorders.

### Background

Acute liver failure (ALF) is an acute syndrome with high mortality, which is characterized by the rapid necrosis and apoptosis of a large number of parenchymal hepatic cells within a short period of time, and the loss of the original liver function. It is accompanied by complications such as disorders of the immune system in the liver, hepatic encephalopathy and possible multi-organ failure.

There are three types of causes of acute liver failure, which are caused by hepatitis viruses such as Hepatitis A, B & E; caused by drug use or poisoning, such as Chinese Herbal Medicine, acetaminophen; other factors such as ischemic hepatocellular injury, hypoxic hepatitis and so on.

At present, the only effective treatment for acute liver failure is liver transplantation, but liver transplantation has many problems and is still not a perfect treatment for acute liver failure, such as the shortage of donor livers, resulting in few patients who can receive liver transplantation in a timely manner, the rejection reaction that still exists after liver transplantation and the need for lifelong immunosuppression and other defects.

As an alternative to liver transplantation, there are currently two strategies: in vitro artificial liver and cell therapy. Cell therapy is a relatively new and simple and feasible therapy, and there have been quite a few reports on animal-level experiments. For example, Mesenchymal Stem Cells (MSCs) transplantation in the treatment of acute liver failure has been proved to have a certain effect, but the molecular mechanism research of MSCs in the treatment of acute liver failure is still limited to the aspect of immune regulation. It is found that the relevant immunomodulatory factors are some known to have immunomodulatory effects and, if used alone, have very limited therapeutic effect on acute liver failure.

Therefore, there is an urgent need in the art to develop new protein drugs with good efficacy for liver immune disorders, such as acute liver failure.

### Summary of the invention

The purpose of the present invention is to provide a new protein drug with good efficacy on liver immune disorders, such as acute liver failure.

The purpose of the present invention is to provide a drug and a therapeutic scheme for treating acute liver failure based on a specific protein CST1.

In a first aspect of the present invention, it provides a use of a *CST1* gene, or a protein or a promoter thereof for preparing a composition or preparation for preventing and/or treating liver immune disorders.

In another preferred embodiment, the liver immune disorders includes liver immune disorders caused by excessive activation of IFN-γ signal.

In another preferred embodiment, the liver immune disorders includes acute liver failure and acute liver injury.

In another preferred embodiment, the composition or preparation is also used for one or more purposes selected from the group consisting of:
(a) increasing the survival rate of a mammal;
(b) significantly lowering levels of alanine aminotransferase, aspartate aminotransferase, and/or blood ammonia in serum;
(c) protecting the function of hepatic parenchymatous cell;
(d) inhibiting the pro-inflammatory activation of IFN-γ on a macrophage;
(e) inhibiting the expression of a large number of ISGs downstream of IFN-γ;
(f) attenuating the efficiency of IFN-γ binding to IFNGR2.

In another preferred embodiment, the mammal includes a human or a non-human mammal.

In another preferred embodiment, the non-human mammal includes a rodent (e.g., mouse, rat, rabbit), and primate (e.g., monkey).

In another preferred embodiment, the promoter refers to a substance that can increase the activity and/or content of the *CST1* gene or a protein thereof in vivo or in vitro; the substance may be a synthetic or natural compound, protein, nucleotide etc.

In another preferred embodiment, the CST1 promoter includes a substance that promotes the expression of CST1.

In another preferred embodiment, the CST1 promoter includes a CST1 protein promoter and/or a CST1 gene promoter.

In another preferred embodiment, the promotion of CST1 expression or activity refers to increasing the expression or activity of CST1 gene or protein by ≥20%, preferably ≥50%, more preferably ≥70%.

In another preferred embodiment, the CST1 promoter is selected from the group consisting of a small molecule compound, a vector expressing CST1, and a combination thereof.

In another preferred embodiment, the vector for expressing CST1 includes a viral vector.

In another preferred embodiment, the viral vector is selected from the group consisting of adeno-associated viral vector, lentiviral vector, and a combination thereof.

In another preferred embodiment, the protein includes a full-length protein or a protein fragment.

In another preferred embodiment, the *CST1* gene or a protein thereof is derived from a mammal, more preferably from a rodent (e.g., mouse, rat), primate and human.

In another preferred embodiment, the *CST1* gene or a protein thereof is derived from an endodermal stem cell or definitive endoderm.

In another preferred embodiment, the CST1 protein further includes a derivative of the CST1 protein.

In another preferred embodiment, the derivative of the CST1 protein includes a modified CST1 protein, a protein molecule whose amino acid sequence is homologous to the natural CST1 protein and has the activity of the natural CST1 protein, a dimer or multimer of the CST1 protein, a fusion protein containing the amino acid sequence of the CST1 protein.

In another preferred embodiment, the modified CST1 protein is a PEGylated CST1 protein.

In another preferred embodiment, the "protein molecule whose amino acid sequence is homologous to the natural CST1 protein and has the activity of the natural CST1 protein" means that a protein molecule whose amino acid sequence has ≥85% homology, preferably ≥90% homology, more preferably ≥95% homology, most preferably ≥98% homology with CST1 protein and has the activity of native CST1 protein.

In another preferred embodiment, the CST1 protein is selected from the group consisting of:
(A) a polypeptide whose amino acid sequence is as shown in SEQ ID NO.: 1;
(B) a CST1 protein derivative or an active fragment thereof formed by the amino acid sequence as shown in SEQ ID NO.: 1 through the substitution, deletion or addition of one or several (usually 1-60, preferably 1-30, more preferably 1-20, most preferably 1-10) amino acid residues;
(C) compared with the amino acid sequence as shown in SEQ ID NO.: 1, a CST1 protein derivative or an active fragment thereof with homology ≥90%, preferably ≥ 95%, more preferably ≥98%, most preferably ≥99%.

In another preferred embodiment, the CST1 gene encodes a CST1 protein.

In another preferred embodiment, the CST1 gene is selected from the group consisting of:
(a) a polynucleotide encoding a polypeptide as shown in SEQ ID NO.: 1;
(b) a polynucleotide whose sequence is shown in SEQ ID NO.: 2;
(c) a polynucleotide in which the homology between the nucleotide sequence and the sequence as shown in SEQ ID NO.:2 is ≥95% (preferably ≥98%), and encoding the polypeptide as shown in SEQ ID NO.:1;
(d) a polynucleotide complementary to any of the polynucleotides of (a)-(c).

In another preferred embodiment, the composition includes a pharmaceutical composition.

In another preferred embodiment, the pharmaceutical composition contains (a) a *CST1* gene, or a protein or a promoter thereof; and (b) a pharmaceutically acceptable carrier.

In another preferred embodiment, the pharmaceutical composition further includes a stem cell that does not express CST1 gene or a protein thereof.

In another preferred embodiment, the stem cell includes an embryonic stem cell and mesenchymal stem cell.

In another preferred embodiment, the stem cell includes a human embryonic stem cell.

In another preferred embodiment, the pharmaceutical composition is liquid, solid, or semi-solid.

In another preferred embodiment, the dosage form of the pharmaceutical composition includes tablets, granules, capsules, oral liquids, or injections.

In another preferred embodiment, in the pharmaceutical composition, the component (a) accounts for 60-99wt%, preferably 75-99wt%, more preferably 80-99wt% of the total weight of the pharmaceutical composition.

In another preferred embodiment, the composition further includes other drugs for preventing and/or treating liver immune disorders.

In another preferred embodiment, the composition or preparation can be used alone or in combination in the prevention and/or treatment of liver immune disorders.

In another preferred embodiment, the use in combination includes: use in combination with other drugs for the prevention and/or treatment of liver immune disorders.

In a second aspect of the present invention, it provides a use of an inhibitor of IFN-γ or a receptor thereof for preparing a composition or preparation for preventing and/or treating liver immune disorders.

In another preferred embodiment, the inhibitor is selected from the group consisting of an antibody, a small molecule compound, and a combination thereof.

In another preferred embodiment, the inhibitor of IFN-γ or a receptor thereof inhibits the binding of IFN-γ to a receptor thereof.

In another preferred embodiment, the inhibitor of IFN-γ or a receptor thereof reduces the binding efficiency of IFN-γ and a receptor thereof.

In another preferred embodiment, the inhibitor of IFN-γ or a receptor thereof reduces the binding efficiency of IFN-γ protein and its receptor protein, and reduces the ratio of said IFN-γ receptor protein to IFN-γ protein.

In another preferred embodiment, the inhibitor of IFN- γ or a receptor thereof reduces the ratio of IFN- y receptor protein to IFN- y protein by ≥30%, preferably ≥50%, more preferably, ≥70%.

In another preferred embodiment, the IFN-γ protein includes a full-length protein or a protein fragment.

In another preferred embodiment, the IFN-γ includes an active fragment of IFN-γ or a derivative thereof.

In another preferred embodiment, the homology between the active fragment or a derivative thereof and IFN-γ is at least 90%, preferably 95%, more preferably 98%, 99%.

In another preferred embodiment, the active fragment or a derivative thereof has at least 80%, 85%, 90%, 95%, 100% of the IFN-γ activity.

In another preferred embodiment, the IFN-γ protein also includes a derivative of IFN-γ protein.

In another preferred embodiment, the derivative of IFN-γ protein includes a modified IFN-γ protein, a protein molecule whose amino acid sequence is homologous to natural IFN-γ protein and has activity of natural IFN-γ protein, a dimer or multimer of IFN-γ protein, a fusion protein containing the amino acid sequence of IFN-γ protein.

In another preferred embodiment, the "protein molecule whose amino acid sequence is homologous to natural IFN-γ protein and has activity of natural IFN-γ protein," means that a protein molecule whose amino acid sequence has ≥ 85% homology, preferably ≥90% homology, more preferably ≥95% homology, most preferably ≥98% homology with IFN-γ protein and has the activity of native IFN-γ protein.

In another preferred embodiment, the IFN-γ is derived from a mammal; preferably, from a human, mouse, rat, or rabbit; more preferably, from a human.

In another preferred embodiment, the receptor for IFN-γ is selected from the group consisting of IFNGR2, IFNGR1, and a combination thereof.

In another preferred embodiment, the receptor for IFN-γ includes a full-length protein or a protein fragment.

In another preferred embodiment, the IFN-γ receptor includes an active fragment of IFN-γ receptor or a derivative thereof.

In another preferred embodiment, the homology between the active fragment or a derivative thereof and the IFN-γ receptor is at least 90%, preferably 95%, more preferably 98%, 99%.

In another preferred embodiment, the active fragment or a derivative thereof has at least 80%, 85%, 90%, 95%, 100% of the IFN-γ activity.

In another preferred embodiment, the IFN-γ receptor also includes a derivative of the IFN-γ receptor.

In another preferred embodiment, the derivative of the IFN-γ receptor includes a modified IFN-γ receptor, a protein molecule whose amino acid sequence is homologous to natural IFN-γ receptor and has activity of natural IFN-γ receptor, a dimer or multimer of IFN-γ receptor, a fusion protein containing the amino acid sequence of IFN-γ receptor.

In another preferred embodiment, the "protein molecule whose amino acid sequence is homologous to natural IFN-γ receptor and has activity of natural IFN-γ receptor" means that a protein molecule whose amino acid sequence has ≥ 85% homology, preferably ≥90% homology, more preferably ≥ 95% homology, most preferably ≥98% homology with IFN-γ receptor and has the activity of native IFN-γ receptor.

In another preferred embodiment, the IFN-γ receptor is derived from a mammal; preferably, from a human, mouse, rat, or rabbit; more preferably, from a human.

In another preferred embodiment, the composition includes a pharmaceutical composition.

In another preferred embodiment, the pharmaceutical composition contains (a) an inhibitor of IFN-γ or a receptor thereof; and (b) a pharmaceutically acceptable carrier.

In another preferred embodiment, the pharmaceutical composition is liquid, solid, or semi-solid.

In another preferred embodiment, the dosage form of the pharmaceutical composition includes tablets, granules, capsules, oral liquids, or injections.

In another preferred embodiment, in the pharmaceutical composition, the component (a) accounts for 60-99wt%, preferably 75-99wt%, more preferably 80-99wt% of the total weight of the pharmaceutical composition.

In another preferred embodiment, the composition further includes other drugs for preventing and/or treating liver immune disorders.

In another preferred embodiment, the composition or preparation can be used alone or in combination in the prevention and/or treatment of liver immune disorders.

In another preferred embodiment, the use in combination includes: use in combination with other drugs for the prevention and/or treatment of liver immune disorders.

In a third aspect of the present invention, it provides a cell preparation, comprising:
(a1) a first pharmaceutical composition containing (a) a first active ingredient, the first active ingredient being the CST1 gene, or a protein or a promoter thereof; or an inhibitor of IFN-γ or a receptor thereof, and a pharmaceutically acceptable carrier;
(a2) an optional second pharmaceutical composition containing (b) a second active ingredient, the second active ingredient being a stem cell that does not express the CST1 gene or a protein thereof; and a pharmaceutically acceptable accepted carrier.

In another preferred embodiment, the first pharmaceutical composition and the second pharmaceutical composition are different pharmaceutical compositions, or the same pharmaceutical composition.

In another preferred embodiment, the stem cell includes an embryonic stem cell and mesenchymal stem cell.

In another preferred embodiment, the stem cell includes a human embryonic stem cell.

In another preferred embodiment, the cell preparation is a liquid preparation.

In another preferred embodiment, the cells in the cell preparation are substantially (≥90%, 95%, 96%, 97%, 98%, 99%, 99.5%, 99.9%) or entirely composed of stem cells that do not express the CST1 gene or a protein thereof.

In another preferred embodiment, in the cell preparation, the component (a1) accounts for 0.5-10wt%, preferably 1-10wt%, more preferably 2-10wt% of the total weight of the cell preparation.

In another preferred embodiment, in the cell preparation, the component (a2) accounts for 90-99wt%, preferably 90-98wt%, more preferably 90-97wt% of the total weight of the cell preparation.

In another preferred embodiment, the weight ratio of the first active ingredient to the second active ingredient is 1:100 to 100:1, preferably 1:10 to 10:1.

In another preferred embodiment, in the cell preparation, the concentration of the component (a1) is 100-1000ug/ml, preferably 200-1000ug/ml, more preferably 300-1000ug/ml.

In another preferred embodiment, in the cell preparation, the concentration of the component (a2) is 1.0×10⁶-1.0×10⁸ cells/ml, preferably 5.0×10⁶-1.0×10⁸ cells/ml, more preferably 1.0×10⁷-1.0×10⁸ cells/ml.

In another preferred embodiment, the carrier is selected from the group consisting of: an infusion agent carrier and/or an injection carrier, preferably, the carrier is one or more carriers selected from the group consisting of: normal saline, glucose saline, and a combination thereof.

In another preferred embodiment, the cell preparation further includes other drugs for preventing and/or treating liver immune disorders.

In a fourth aspect of the present invention, it provides a kit, comprising:
(i) a first container, and the active ingredient (a1) the CST1 gene, or a protein thereof, or a promoter thereof; or an inhibitor of IFN-γ or a receptor thereof, located in the first container, or a medicament containing the active ingredient (a); and
(ii) an optional second container, and the active ingredient (a2) a stem cell that does not express the CST1 gene or a protein thereof located in the second container, or a medicament containing active ingredient (a2).

In another preferred embodiment, the first container and the second container are the same or different containers.

In another preferred embodiment, the medicament located in the first container is a single preparation containing CST1 gene, or a protein or a promoter thereof.

In another preferred embodiment, the medicament located in the second container is a single preparation containing a stem cell that does not express CST1 gene or a protein thereof.

In another preferred embodiment, the dosage form of the medicament is an oral dosage form or an injection dosage form.

In another preferred embodiment, the kit further contains instructions, which describe the instructions for co-administering the active ingredient (a1) and the active ingredient (a2) to prevent and/or treat liver immune disorders.

In another preferred embodiment, the dosage form of the preparation containing the active ingredient (a1) CST1 gene, or a protein or a promoter thereof; or an inhibitor of IFN-γ or a receptor thereof, or that of the preparation containing other drugs for preventing and/or treating liver immune disorders includes capsules, tablets, suppositories, or intravenous injections, respectively.

In another preferred embodiment, in the preparation containing the active ingredient (a1) CST1 gene, or a protein or a promoter thereof; or an inhibitor of IFN-γ or a receptor thereof, the concentration of the CST1 gene, or a protein or a promoter thereof; or the inhibitor of IFN-γ or a receptor thereof is 0.1-100 mg/kg body weight, preferably 0.1-50 mg/kg body weight, more preferably 1-20 mg /kg body weight.

In another preferred embodiment, in the preparation containing the active ingredient (a2) a stem cell that does not express the CST1 gene or a protein thereof, the concentration of the stem cell that does not express the CST1 gene or a protein thereof is 0.1-100 mg/kg body weight, preferably 0.1-50mg/kg body weight, more preferably 1-20mg/kg body weight.

In a fifth aspect of the present invention, it provides a method for screening a potential therapeutic agent for preventing and/or treating liver immune disorders, comprising:
(a) in a test group, in a culture system, in the presence of a test compound, culturing a cell expressing a *CST1* gene for a period of time T1, and detecting the expression level E1 of the *CST1* gene in the culture system of the test group;
   and in a control group without the test compound and other conditions being the same, detecting the expression level E2 of the CST1 gene in the culture system of the control group; and
(b) comparing E1 and E2, if E1 is significantly higher than E2, indicating that the test compound is a potential therapeutic agent for liver immune disorders.

In another preferred embodiment, the "significantly higher" refers to E1/E2≥2, preferably, ≥3, more preferably, ≥4.

In another preferred embodiment, the cell includes a human embryonic stem cell and human endodermal stem cell.

In another preferred embodiment, the cell is a cell cultured in vitro.

In another preferred embodiment, the method is non-diagnostic and non-therapeutic.

In another preferred embodiment, the method includes step (c): administering the potential therapeutic agent identified in step (a) to a mammal, thereby determining its effect on the liver immune disorders in a mammal.

In another preferred embodiment, the mammal includes a human or a non-human mammal.

In another preferred embodiment, the non-human mammal includes a rodent and primate, preferably mouse, rat, rabbit, and monkey.

In a sixth aspect of the present invention, it provides a method for screening a potential therapeutic agent for preventing and/or treating liver immune disorders, comprising:
(a) in a test group, in a culture system, in the presence of a test compound, culturing a cell for a period of time T1, and detecting the binding of the IFN-γ to its receptors in the culture system of the test group;
   and in a control group without the test compound and with the same other conditions, detecting the binding of the IFN-γ to its receptor in the culture system of the control group;
(b) if the binding efficiency E1 of the IFN-γ to its receptor in the test group is significantly lower than the binding efficiency E2 of the IFN-γ to its receptor in the control group, indicating that the test compound is a candidate compound.

In another preferred embodiment, the method includes step (b): administering the candidate compound determined in step (a) to a mammal, thereby determining its effect on liver immune disorders in a mammal.

In another preferred embodiment, the mammal includes a human or a non-human mammal.

In another preferred embodiment, the non-human mammal includes a rodent and primate, preferably a mouse, rat, rabbit, and monkey.

In another preferred embodiment, the "significantly lower than" refers to E1/E2≤ 1/2, preferably, ≤ 1/3, more preferably≤1/4.

In another preferred embodiment, the method is non-diagnostic and non-therapeutic.

In another preferred embodiment, the cell includes a human embryonic stem cell, human endodermal stem cell, and human mesenchymal stem cell.

In another preferred embodiment, the cell is a cell cultured in vitro.

In a seventh aspect of the present invention, it provides a method for preventing and/or treating liver immune disorders, comprising the steps of:
Administering a CST1 gene, or a protein or a promoter thereof or an inhibitor of IFN-γ or a receptor thereof, the cell preparation according to the second aspect of the present invention, or the kit according to the third aspect of the present invention to a subject in need.

In another preferred embodiment, the administration comprises oral administration.

In another preferred embodiment, the subject includes a human or a non-human mammal.

In another preferred embodiment, the non-human mammal includes a rodent and primate, preferably a mouse, rat, rabbit, and monkey.

In another preferred embodiment, the administration dosage of the CST1 gene, or a protein or a promoter thereof; or the inhibitor of IFN-γ or a receptor thereof is 0.1-100 mg/kg body weight, preferably 1-50 mg/kg body weight, more preferably 5-20 mg/kg body weight.

In another preferred embodiment, the administration frequency of the CST1 gene, or a protein or a promoter thereof; or the inhibitor of IFN-γ or a receptor thereof is injection once every 7-30 days, preferably once every 14-30 days.

In another preferred embodiment, the administration time of the CST1 gene, or a protein or a promoter thereof; or the inhibitor of IFN-γ or a receptor thereof is injection once every 7 days to 30 days, preferably once every 14 to 30 days.

It should be understood that, within the scope of the present invention, the technical features specifically described above and below (such as in the Examples) can be combined with each other, thereby constituting a new or preferred technical solution which needs not be described one by one.

### DESCRIPTION OF THE DRAWINGS

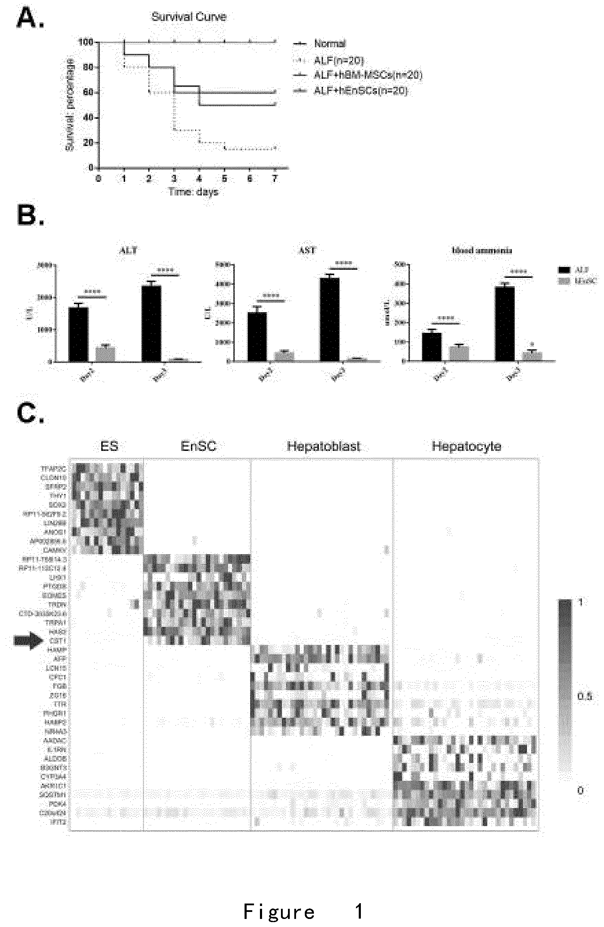

Figure 1 shows that in the cell line hEnSCs expressing CST1, transplantation of hEnSCs into the liver of acute liver failure rats can effectively treat acute liver failure. It can greatly improve the survival rate of rats and protect liver function. Compared with cell lines known to be effective in acute liver failure but not expressing CST1, mesenchymal stem cells, hEnSCs have comparable or even better efficacy.
A. One-week survival curve of hEnSC transplantation in the treatment of acute liver failure in rats, 20 rats per group. Groups represented by legend: Normal: healthy rats; ALF: rats with acute liver failure; ALF+hBM-MSCs: rats transplanted with human bone marrow-derived mesenchymal stem cells for the treatment of acute liver failure; ALF+hEnSC: rats transplanted with hEnSC cell treatment.
B. Changes of liver function indexes on days 2 and 3 in rats transplanted with hEnSCs to treat acute liver failure. ALT: alanine transaminase; AST: aspertate aminotransferase; Blood Ammonia: Ammo.
C. Single-cell sequencing heatmap (Heatmap). The sequencing library construction method is Smart-seq2, and the sequencing platform is the Illumina next-generation sequencing platform. The gene pointed to by the red arrow is what we believe to be a potential key factor, CST1.

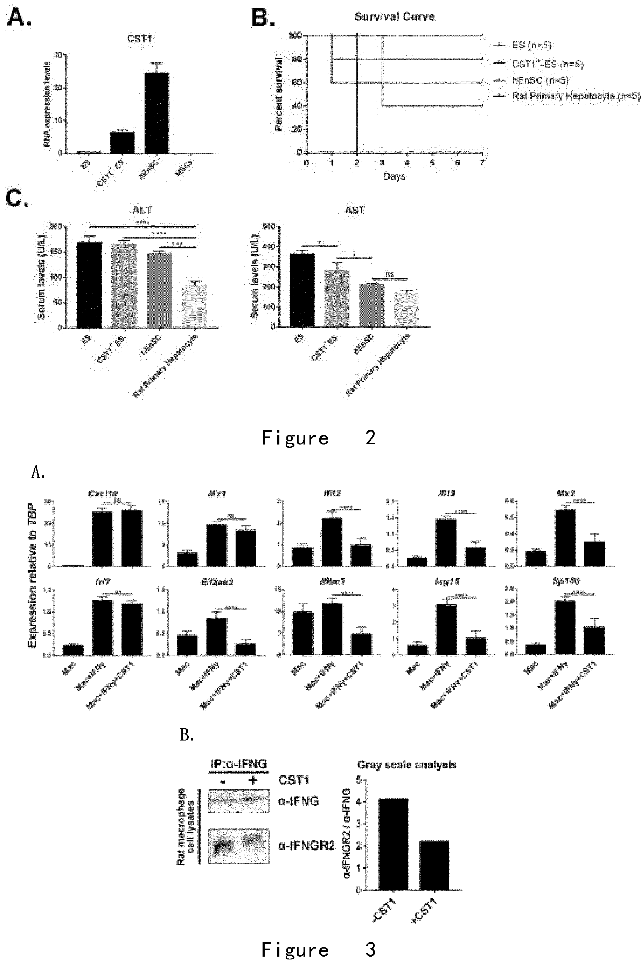

Figure 2 shows that the overexpression of CST1 in an embryonic stem cell, a cell line that does not express CST1 and cannot treat acute liver failure, can enable it to obtain a certain therapeutic effect in the treatment of acute liver failure. It is manifested in the improvement of survival rate and the protection of liver function.
A. RNA expression levels of CST1.
B. Survival curves of acute liver failure rats transplanted with different types of cells within one week, 5 rats in each group. Rat Primary Hepatocyte: Rat-derived hepatocytes.
C. The indexes of serum ALT and AST on the third day of acute liver failure rats transplanted with different types of cells. The significance between different groups is indicated by the number of "^{∗}", and "ns" means no significance.

Figure 3 shows that CST1 protein can significantly reduce the expression of IFN-γ-activated downstream genes ISGs (Interferon stimulated genes, interferon-activated genes) in macrophages (Mac, Macrophage). The reason for this inhibition is that CST1 can interfere with IFN-γ binding to its receptor IFNGR2, thereby blocking the expression of ISGs genes that further activate the downstream of IFNGR2.
A. The expression changes of ISGs in macrophages after adding CST1. When adding IFN-γ to activate the expression of downstream ISGs in macrophages cultured in vitro, the addition of CST1 can significantly inhibit the increased expression of ISGs.
B. Co-immunoprecipitation assay to identify the effect of CST1 on the binding efficiency of IFN-γ and its receptor IFNGR2. IFN-γ antibody is used as an antibody for co-immunoprecipitation, and simultaneously separates IFN-γ and IFNGR2 bound to it. When CST1 is added to this separation system, the amount of IFNGR2 bound to IFN-γ is reduced, and the reaction is shown that reduced IFNGR2/IFN-y ratio detected in western blot, indicating that CST1 affects the binding of IFN-γ and IFNGR2, and reduces the binding efficiency of the two.

### Detailed Description

After extensive and in-depth research and extensive screening, the present inventors have unexpectedly discovered for the first time that CST1 protein gene, or its protein, or its promoter; or an inhibitor of IFN-γ or its receptor can significantly prevent and/or treat liver immune disorders, in addition, the present invention has also unexpectedly found that the CST1 protein gene, or its protein, or its promoter can also (a) improve the survival rate of mammals; and/or (b) significantly reduce serum alanine transaminase, aspartate aminotransferase, and/or blood ammonia levels; and/or (c) protect the function of hepatic parenchymatous cell; and/or (d) inhibit the pro-inflammatory activation of IFN-γ on macrophages; and/or (e) inhibit the expression of a large number of ISGs downstream of IFN-γ; and/or (f) attenuate the efficiency of IFN-γ binding to IFNGR2. In addition, the present invention has also unexpectedly found that overexpression of CST1 can make ineffective cells obtain a certain therapeutic effect, which shows that CST1 protein itself and cell therapy based on CST1 overexpression have great potential for the treatment of acute liver failure. On this basis, the present inventors have completed the present invention.

### Terms

Unless otherwise defined, all technical and scientific terms used herein have the same meaning as commonly understood by one of ordinary skill in the art to which this invention belongs.

As used herein, when used in reference to a specifically recited value, the term "about" means that the value may vary by no more than 1% from the recited value. For example, as used herein, the expression "about 100" includes all values between 99 and 101 (e.g., 99.1, 99.2, 99.3, 99.4, etc.).

As used herein, the terms "containing" or "comprising (including)" can be open, semi-closed, and closed. In other words, the term also includes "consisting essentially of," or "consisting of."

As used herein, the term "administration" refers to the physical introduction of a product of the present invention into a subject using any of a variety of methods and delivery systems known to those of skill in the art, including intravenous, intramuscular, subcutaneous, intraperitoneal, spinal or other parenteral routes of administration, for example by injection or infusion.

### CST1 protein and an encoding nucleic acid thereof

As used herein, the terms "CST1 protein", "Cystatin SN protein", " Cysteine protease inhibitor SN" are used interchangeably.

The present invention relates to a CST1 protein and a variant thereof. In a preferred embodiment of the present invention, the amino acid sequence of the CST1 protein is shown in SEQ ID NO.: 1. The CST1 protein or its promoter of the present invention can (a) prevent and/or treat liver immune disorders; and/or (b) improve the survival rate of mammals; and/or (c) significantly reduce serum alanine transaminase, aspartate aminotransferase, and/or blood ammonia levels.

The present invention also includes polypeptides or proteins with the same or similar functions that have 50% or more (preferably more than 60%, more than 70%, more than 80%, more preferably more than 90%, more preferably more than 95%, most preferably more than 98%, such as 99%) homology with the sequence as shown in SEQ ID NO.: 1 of the present invention

Wherein, SEQ ID NO.: 1 is a human CST1 protein, and the accession number is NM_001898.2.

The "same or similar function" mainly refers to: "(a) preventing and/or treating liver immune disorders; and/or (b) improving the survival rate of mammals; and/or (c) significantly reducing serum alanine transaminase, aspartate aminotransferase, and/or blood ammonia levels".

The protein of the present invention can be a recombinant protein, a natural protein, or a synthetic protein. The proteins of the invention may be naturally purified products, or chemically synthesized products, or produced using recombinant techniques from prokaryotic or eukaryotic hosts (e.g., bacterial, yeast, higher plant, insect and mammalian cells). Depending on the host used in the recombinant production protocol, the proteins of the invention may be glycosylated or may be non-glycosylated. The proteins of the invention may or may not also include an initial methionine residue.

The present invention also includes CST1 protein fragments and analogs having CST1 protein activity. As used herein, the terms "fragment" and "analog" refer to proteins that retain substantially the same biological function or activity of the native CST1 protein of the invention.

A mutant protein fragment, derivative or analog of the present invention may be (i) a mutant protein having one or more conservative or non-conservative amino acid residues (preferably conservative amino acid residues) substituted, and such substituted amino acid residues may or may not be encoded by the genetic code, or (ii) a mutant protein with a substitution group in one or more amino acid residues, or (iii) a mutant protein formed by fusion of a mature mutant protein with another compound (such as compounds that prolong the half-life of mutant protein, such as polyethylene glycol), or (iv) a mutant protein formed by fusing an additional amino acid sequence to this mutant protein sequence (such as a leader sequence or secretory sequence or sequence used to purify the mutant protein or proprotein sequence, or fusion protein with antigenic IgG fragment). sequence or proprotein sequence, or a fusion protein formed with an antigen IgG fragment). These fragments, derivatives and analogs are well known to those skilled in the art in light of the teachings herein. In the present invention, conservatively substituted amino acids are preferably produced by amino acid substitutions according to Table 1.

**Table I**

| initial residue | representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gin (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

The present invention also includes polypeptides or proteins with the same or similar functions that have 50% or more (preferably more than 60%, more than 70%, more than 80%, more preferably more than 90%, more preferably more than 95%, most preferably more than 98%, such as 99%) homology with the native CST1 protein of the present invention. In a protein variant, the derivative sequence can be obtained by several (usually 1-60, preferably 1-30, more preferably 1-20, most preferably 1-10) substitutions, deletions or additions of at least one amino acid, as well as the addition of one or several (usually within 20, preferably within 10, more preferably within 5) amino acids to the C-terminus and/or N-terminus. For example, in the protein, substitution with amino acids with close or similar properties usually does not change the function of the protein, and adding one or more amino acids to the C-terminus and/or \-terminus usually does not change the function of the protein. The present invention includes the difference between the natural CST1 protein analog and the natural CST1 protein, which can be the difference in amino acid sequence, or the difference in modified form that does not affect the sequence, or both. Analogs of these proteins include natural or induced genetic variants. Induced variants can be obtained by a variety of techniques, such as random mutagenesis by radiation or exposure to mutagens, site-directed mutagenesis or other known molecular biology techniques. Analogs also include analogs with residues other than natural L-amino acids (e.g., D-amino acids), as well as analogs with non-naturally occurring or synthetic amino acids (e.g., beta, gamma-amino acids). It should be understood that the proteins of the present invention are not limited to the representative proteins exemplified above.

Modified (usually without altering the primary structure) forms include chemically derivatized forms of the protein in vivo or in vitro such as acetoxylation or carboxylation. Modifications also include glycosylation, such as glycosylation modifications carried out in protein synthesis and processing. This modification can be accomplished by exposing the protein to enzymes that perform glycosylation, such as mammalian glycosylases or deglycosylases. Modified forms also include sequences with phosphorylated amino acid residues (e.g., phosphotyrosine, phosphoserine, phosphothreonine). In addition, the muteins of the present invention can also be modified. Modified (usually without altering the primary structure) forms include chemically derivatized forms of muteins such as acetylated or carboxylated in vivo or in vitro. Modifications also include glycosylation, such as those resulting from glycosylation modifications in the synthesis and processing of the mutein or in further processing steps. This modification can be accomplished by exposing the muteins to enzymes that perform glycosylation, such as mammalian glycosylases or deglycosylases. Modified forms also include sequences with phosphorylated amino acid residues (e.g., phosphotyrosine, phosphoserine, phosphothreonine). Also included are mutant proteins that have been modified to increase their resistance to proteolysis or to optimize solubility.

The present invention also provides a polynucleotide sequence encoding the CST1 protein. The polynucleotides of the present invention may be in the form of DNA or RNA. DNA forms include: DNA, genomic DNA or synthetic DNA, DNA can be single-stranded or double-stranded. Polynucleotides encoding mature polypeptides include: coding sequences encoding only the mature polypeptide; coding sequences and various additional coding sequences for the mature polypeptide; coding sequences (and optional additional coding sequences) for the mature polypeptide, and non-coding sequences. The term "polynucleotide encoding a polypeptide" may include a polynucleotide encoding the polypeptide or a polynucleotide that also includes additional coding and/or non-coding sequences. The present invention also relates to variants of the above-mentioned polynucleotides, which encode fragments, analogs and derivatives of polypeptides having the same amino acid sequence as the present invention. Variants of this polynucleotide can be naturally occurring allelic variants or non-naturally occurring variants. These nucleotide variants include substitution variants, deletion variants, and insertion variants. As known in the art, an allelic variant is an alternative form of a polynucleotide, which may be a substitution, deletion or insertion of one or more nucleotides that does not substantially alter the function of the encoded polypeptide.

In a preferred example of the present invention, the DNA sequence encoding the human CST1 protein encodes the ECM1 protein as shown in SEQ ID NO.: 1, and the polynucleotide sequence encoding the CST1 protein is shown in SEQ ID NO.: 2.

Wherein, SEQ ID NO.: 2 is the nucleotide sequence of the human CST1 gene.

Based on the nucleotide sequences as described herein, those skilled in the art can readily prepare the encoding nucleic acids of the present invention by various known methods. These methods are such as but not limited to: PCR, artificial DNA synthesis, etc. For specific methods, please refer to J. Sambrook, "Molecular Cloning Experiment Guide". As an embodiment of the present invention, the coding nucleic acid sequence of the present invention can be constructed by the method of synthesizing nucleotide sequences by segment and then performing overlap extension PCR.

The present invention also relates to polynucleotides which hybridize to the above-mentioned sequences and have at least 50%, preferably at least 70%, more preferably at least 80% identity between the two sequences. In particular, the present invention relates to polynucleotides that hybridize under strict conditions (or stringent conditions) to the polynucleotides of the present invention. In the present invention, "stringent conditions" refer to: (1) hybridization and elution at lower ionic strength and higher temperature, such as 0.2×SSC, 0.1% SDS, 60°C; or (2) A denaturant is added during hybridization, such as 50% (v/v) formamide, 0.1% calf serum/0.1% Ficoll, 42°C, etc.; or (3) Hybridization occurs only when the identity between the two sequences is at least 90% or more, more preferably 95% or more.

The proteins and polynucleotides of the present invention are preferably provided in isolated form, more preferably, purified to homogeneity.

The full-length sequence of the polynucleotide of the present invention can usually be obtained by PCR amplification method, recombinant method or artificial synthesis method. For the PCR amplification method, primers can be designed according to the relevant nucleotide sequences disclosed in the present invention, especially the open reading frame sequences, and the relevant sequences are obtained by amplifying the commercially available cDNA library or the cDNA library prepared by conventional methods known to those skilled in the art as templates. When the sequence is long, it is often necessary to perform two or more PCR amplifications, and the amplified fragments are then spliced together in the correct order.

Once the relevant sequences have been obtained, recombinant methods can be used to obtain the relevant sequences in bulk. This is usually done by cloning it into a vector, transferring it into a cell, and isolating the relevant sequence from the propagated host cell by conventional methods.

In addition, synthetic methods can also be used to synthesize the relevant sequences, especially when the fragment length is short. Often, fragments of very long sequences are obtained by synthesizing multiple small fragments followed by ligation.

At present, the DNA sequences encoding the proteins of the present invention (or fragments thereof, or derivatives thereof) can be obtained entirely by chemical synthesis. This DNA sequence can then be introduced into various existing DNA molecules (or e.g., vectors) and cells known in the art. In addition, mutations can also be introduced into the protein sequences of the invention by chemical synthesis.

Methods of amplifying DNA/RNA using PCR techniques are preferred for obtaining the polynucleotides of the present invention. In particular, when it is difficult to obtain a full-length cDNA from the library, the RACE method (RACE-rapid amplification of cDNA ends) can be preferably used, and the primers for PCR can be appropriately selected according to the sequence information of the present invention disclosed herein, and can be synthesized by conventional methods. Amplified DNA/RNA fragments can be isolated and purified by conventional methods such as by gel electrophoresis.

### CST1 promoter

In the present invention, CST1 promoters include substances capable of increasing the activity and/or content of the *CST1* gene or its protein in vivo or in vitro.

Among them, the expression of CST1 can be increased by the following methods: tissue itself secretes a large amount of CST1 protein or artificially overexpresses CST1 protein, or artificially delivers CST1 protein (for example, using a viral vector, such as adeno-associated virus vector) or CST1 promoters.

In the present invention, the CST1 promoter is not particularly limited, as long as it can promote the expression of CST11 or enhance the activity of the CST1 protein, it is within the protection scope of the present invention.

In a preferred embodiment, the CST1 promoter comprises a small molecule compound.

### IFN-γ or its receptor

In the present invention, the terms "IFN-γ or its receptor protein" and "IFN-γ or its receptor polypeptide" are used interchangeably, and both refer to a protein or polypeptide having the amino acid sequence of IFN-γ or its receptor. They include IFN-γ or its receptor protein with or without the starting methionine. In addition, the term also includes full-length IFN-γ or its receptor and fragments thereof. The IFN-γ or its receptor protein referred to in the present invention includes its complete amino acid sequence, its secreted protein, its mutants and its functionally active fragments.

IFN-γ or its receptors are type II interferon and type II interferon binding receptors on the cell surface, including, but not limited to, IFN-γ, IFNGR1, IFNGR2.

In a preferred embodiment, the IFN-γ receptor is selected from the group consisting of IFNGR2, IFNAR1, IFNAR2 (wherein IFNAR1, IFNAR2 are type I interferon receptors), and a combination thereof.

In the present invention, IFN-γ is human IFN-γ protein, and the accession number is P01579.

The IFN-γ receptor is a human IFN-γ receptor protein, and the accession numbers are as follows respectively:
IFNGR2: P38484;
IFNGR1: P15260.

When an amino acid fragment of IFN-γ or its receptor is obtained, a nucleic acid sequence encoding it can be constructed based on it, and a specific probe can be designed based on the nucleotide sequence. The full-length nucleotide sequence or its fragment can usually be obtained by PCR amplification method, recombinant method or artificial synthesis method. For the PCR amplification method, primers can be designed according to the nucleotide sequences of IFN-γ or its receptors disclosed in the present invention, especially the open reading frame sequences, and use a commercially available cDNA library or a cDNA library prepared by conventional methods known to those skilled in the art as a template to amplify the relevant sequences. When the sequence is long, it is often necessary to perform two or more PCR amplifications, and then splicing the amplified fragments together in the correct order.

Once the relevant sequences have been obtained, recombinant methods can be used to obtain the relevant sequences in bulk. This is usually done by cloning it into a vector, transferring it into a cell, and isolating the relevant sequence from the propagated host cell by conventional methods.

In addition, synthetic methods can also be used to synthesize the relevant sequences, especially when the fragment length is short. Often, fragments of very long sequences are obtained by synthesizing multiple small fragments followed by ligation.

At present, the DNA sequences encoding the proteins of the present invention (or fragments or derivatives thereof) can be obtained completely by chemical synthesis. This DNA sequence can then be introduced into various existing DNA molecules (e.g., vectors) and cells known in the art.

The polynucleotide sequences of the present invention can be used to express or produce recombinant DKK1 polypeptides by conventional recombinant DNA techniques. Generally there are the following steps:
(1) Transform or transduce a suitable host cell with the polynucleotide (or variant) encoding human IFN-γ or its receptor polypeptide of the present invention, or with a recombinant expression vector containing the polynucleotide;
(2) Host cells cultured in a suitable medium;
(3) Separation and purification of proteins from culture medium or cells.

In the present invention, IFN-γ or its receptor polynucleotide sequence can be inserted into a recombinant expression vector. In short, any plasmid and vector can be used as long as it is replicable and stable in the host. An important feature of expression vectors is that they typically contain an origin of replication, a promoter, marker genes and translational control elements.

Methods well known to those skilled in the art can be used to construct expression vectors containing DNA sequences encoding IFN-γ or its receptor and appropriate transcriptional/translational control signals. These methods include in vitro recombinant DNA technology, DNA synthesis technology, in vivo recombinant technology, and the like. The DNA sequence can be operably linked to an appropriate promoter in an expression vector to direct mRNA synthesis. Expression vectors also include a ribosome binding site for translation initiation and a transcription terminator.

In addition, the expression vector preferably contains one or more selectable marker genes to provide phenotypic traits for selection of transformed host cells, such as dihydrofolate reductase for eukaryotic cell culture, neomycin resistance, and green fluorescent protein (GFP), or for tetracycline or ampicillin resistance in E. coli.

Vectors comprising the appropriate DNA sequences as described above, together with appropriate promoter or control sequences, can be used to transform appropriate host cells so that they can express the protein.

Host cells can be prokaryotic cells, such as bacterial cells; or lower eukaryotic cells, such as yeast cells; or higher eukaryotic cells, such as mammalian cells. Representative examples are: Escherichia coli, bacterial cells of the genus Streptomyces; fungal cells such as yeast; plant cells; insect cells; animal cells and the like.

Transformation of host cells with recombinant DNA can be performed using conventional techniques well known to those skilled in the art. When the host is a prokaryotic organism such as E. coli, competent cells capable of uptake of DNA can be harvested after exponential growth phase and treated with the CaCl₂ method using procedures well known in the art. Another way is to use MgCl₂. If desired, transformation can also be performed by electroporation. When the host is a eukaryotic organism, the following DNA transfection methods can be used: calcium phosphate co-precipitation method, conventional mechanical methods such as microinjection, electroporation, liposome packaging, etc.

The obtained transformants can be cultured by conventional methods to express the polypeptides encoded by the genes of the present invention. The medium used in the culture can be selected from various conventional media depending on the host cells used. Cultivation is carried out under conditions suitable for growth of the host cells. After the host cells have grown to an appropriate cell density, the selected promoter is induced by an appropriate method (eg, temperature switching or chemical induction), and the cells are cultured for an additional period of time.

The recombinant polypeptide in the above method can be expressed intracellularly, or on the cell membrane, or secreted outside the cell. If desired, recombinant proteins can be isolated and purified by various isolation methods utilizing their physical, chemical and other properties. These methods are well known to those skilled in the art. Examples of these methods include, but are not limited to: conventional renaturation treatment, treatment with protein precipitants (salting-out method), centrifugation, osmotic disruption, ultratreatment, ultracentrifugation, molecular sieve chromatography (gel filtration), adsorption chromatography, ion exchange chromatography, high performance liquid chromatography (HPLC) and various other liquid chromatography techniques and combinations of these methods.

### Inhibitor of IFN-γ or its receptor and pharmaceutical composition

Using the protein of the present invention, through various conventional screening methods, substances that interact with IFN-γ or its receptors, especially inhibitors and the like, can be screened.

The IFN-γ or its receptor inhibitor (or antagonist) useful in the present invention includes any substance that can inhibit the expression and/or activity of IFN-γ or its receptor gene or its encoded protein.

In the present invention, IFN-γ or its receptor inhibitors also include inhibitors that inhibit the binding of IFN-γ to its receptors (including inhibitors that reduce the binding efficiency of IFN-γ to its receptors or inhibitors that reduce the ratio of IFN-γ protein to receptor protein).

For example, the inhibitors of IFN-γ and its receptors include small molecule compounds, antibodies to IFN-γ and its receptors, antisense RNA, siRNA, shRNA, miRNA, or nucleic acid of IFN-γ and its receptor or activity inhibitors of IFN-γ and its receptors.

In a preferred embodiment, the methods and steps for inhibiting IFN-γ and its receptor include neutralizing its protein with antibodies against IFN-γ and its receptor, gene silencing of IFN-γ and its receptors is performed using shRNA or siRNA or CRISPR reagents carried by viruses (such as adeno-associated virus)..

The inhibition rate of IFN-γ and its receptor is generally at least 50% inhibition, preferably 60%, 70%, 80%, 90%, 95% inhibition, the inhibition rate of IFN-γ and its receptors can be controlled and detected based on conventional techniques, such as flow cytometry, fluorescence quantitative PCR or Western blot.

The inhibitors of IFN-γ and its receptor proteins of the present invention (including antibodies, small molecule compounds, antisense nucleic acids, CRISPR reagents and other inhibitors), when administered therapeutically, the expression and/or activity of IFN-γ and its receptor proteins can be inhibited, or the binding of IFN-γ to its receptors can be inhibited, or the efficiency of IFN-γ binding to its receptors can be reduced , or reduce the ratio of IFN-γ protein to receptor protein, thereby preventing and/or treating liver immune disorders. Generally, these materials can be formulated in a non-toxic, inert and pharmaceutically acceptable aqueous carrier medium, usually at a pH of about 5-8, preferably at a pH of about 6-8, although pH can vary depending on the nature of the substance being formulated and the condition being treated. The formulated pharmaceutical compositions can be administered by conventional routes, including (but not limited to): topical, intramuscular, intraperitoneal, intravenous, subcutaneous, intracutaneous, topical administration, and infusion of autologous cells after extraction and culture.

The present invention also provides a pharmaceutical composition comprising a safe and effective amount of the inhibitor of the present invention (such as an antibody, a compound, a CRISPR reagent, an antisense sequence (such as siRNA), or an inhibitor) and a pharmaceutically acceptable carrier or excipient. Such carriers include, but are not limited to, saline, buffers, dextrose, water, glycerol, ethanol, and combinations thereof. The drug formulation should match the mode of administration. The pharmaceutical composition of the present invention can be prepared in the form of injection, for example, prepared by conventional methods with physiological saline or an aqueous solution containing glucose and other adjuvants. Pharmaceutical compositions, such as tablets and capsules, can be prepared by conventional methods. Pharmaceutical compositions such as injections, solutions, tablets and capsules are preferably manufactured under sterile conditions. The active ingredient is administered in a therapeutically effective amount, e.g., about 1 microgram to 10 mg/kg body weight per day.

### stem cell

In the present invention, the stem cells of the present invention are stem cells that do not express the CST1 gene or its protein.

In a preferred embodiment, the stem cells of the present invention include, but are not limited to, embryonic stem cells and mesenchymal stem cells.

### Pharmaceutical composition (cell preparation)

The present invention also provides a pharmaceutical composition, which contains an effective amount of (a) CST1 gene, or its protein or its promoter or IFN-γ or its receptor inhibitor; (b) optionally a stem cell that does not express the CST1 gene or its protein, and a pharmaceutically acceptable carrier.

Generally, components (a) and (b) can be formulated in a non-toxic, inert and pharmaceutically acceptable aqueous carrier medium, such as physiological saline, wherein the pH is usually about 5-8, preferably, the pH is about 7-8.

As used herein, the term "effective amount" or "effective dose" refers to an amount that produces function or activity in humans and/or animals and is acceptable to humans and/or animals. In a preferred embodiment of the present invention, the effective amount of the component (a) is: 100-1000ug/ml, preferably 200-1000ug/ml, more preferably 300-1000ug/ml; the effective amount of the component (b) is 1.0×10⁶-1.0×10⁸ cells/ml, preferably 5.0×10⁶-1.0×10⁸ cells/ml, more preferably 1.0×10⁷-1.0×10⁸ cells/ml. Preferably, the effective amounts of components (a) and (b) are injected at one time.

As used herein, a "pharmaceutically acceptable" ingredient is one that is suitable for use in humans and/or mammals without undue adverse side effects (e.g., toxicity, irritation, and allergic reaction), i.e., a substance with a reasonable benefit/risk ratio. The term "pharmaceutically acceptable carrier" refers to a carrier for administration of a therapeutic agent, including. In the present invention, the pharmaceutically acceptable carrier that can be used is not particularly limited, and can be one or more compatible solid or liquid fillers or gel substances, which are suitable for human use and must have sufficient purity and sufficiently low toxicity. "Compatibility" as used herein means that the components of the composition can be intermixed with the adipose mesenchymal progenitor cells of the present invention without significantly reducing their therapeutic effect. Examples of pharmaceutically acceptable carrier moieties of the present invention are physiologically acceptable sterile aqueous or non-aqueous solutions, dispersions, suspensions or emulsions, and sterile powders for reconstitution into sterile injectable solutions or dispersions, suitable aqueous and non-aqueous carriers, diluents, solvents or excipients including water, ethanol, polyols and suitable mixtures thereof. In addition to the above-mentioned conventional vectors, optimized vectors can also be designed according to the properties of adipose mesenchymal progenitor cells. The carrier is preferably an infusion carrier and/or an injection carrier.

The pharmaceutical composition of the present invention contains safe and effective amounts of components (a) and (b), and a pharmaceutically acceptable carrier. Such carriers include, but are not limited to, saline, buffers, dextrose, water, glycerol, ethanol, and combinations thereof. Usually, the pharmaceutical preparation should match the mode of administration, and the pharmaceutical composition of the present invention can be prepared in the form of injection, for example, by using normal saline or an aqueous solution containing glucose and other adjuvants by conventional methods. The pharmaceutical compositions are preferably manufactured under sterile conditions. The amount of active ingredient administered is a therapeutically effective amount. The pharmaceutical preparation of the present invention can also be made into a sustained-release preparation.

The effective amounts of components (a) and (b) of the present invention may vary depending on the mode of administration, the severity of the disease to be treated, and the like. Selection of the preferred effective amount can be determined by one of ordinary skill in the art based on various factors (e.g., through clinical trials). The factors include, but are not limited to: the pharmacokinetic parameters such as bioavailability, metabolism, half-life, etc.; the severity of the disease to be treated by the patient, the patient's weight, the patient's immune status, the route of administration, and the like.

The pharmaceutical composition of the present invention is preferably an intravenous injection agent. In another preferred example, in the intravenous injection reagent, the concentration of component (a) is: 100-1000ug/ml, preferably 200-1000ug/ml, more preferably 300-1000ug/ml, the effective amount of the component (b) is 1.0×10⁶-1.0×10⁸ cells/ml, preferably 5.0×10⁶-1.0×10⁸ cells/ml, more preferably 1.0×10⁷-1.0×10⁸ cells/ml. The injection method of the pharmaceutical composition is not particularly limited, and it can be a single injection preparation or a combination of multiple injection preparations. In a preferred embodiment of the present invention, the pharmaceutical composition is a single injection.

In the present invention, the pharmaceutical composition is preferably an intravenous injection preparation.

### treatment method

The present invention also provides a method for (a) preventing and/or treating liver immune disorders; and/or (b) improving survival in mammals; and/or (c) significantly reducing serum alanine aminotransferase, aspartate aminotransferase, and/or blood ammonia levels; and/or (c) protecting function of hepatic parenchymatous cells; and/or (d) inhibiting the pro-inflammatory activation of IFN-γ on macrophages; and/or (e) inhibiting the expression of a large number of ISGs downstream of IFN-γ; and /or (f) attenuating the efficiency of IFN-γ binding to IFNGR2, comprising administering to a mammal an effective amount of the active ingredient (a) the CST1 gene, or its protein or its promoter; or an inhibitor of IFN-γ or its receptor, or administering a pharmaceutical composition containing the active ingredient (a); and optionally (b) stem cells that do not express the CST1 gene or its protein, or administering a pharmaceutical composition containing the active ingredient (b).

When the active ingredient of the present invention is used for the above-mentioned purposes, it can be mixed with one or more pharmaceutically acceptable carriers or excipients, such as solvents, diluents, etc., and can be administered orally in the form of tablets, pills, capsules, dispersible powders, granules or suspensions (containing e.g. about 0.05-5% suspending agent), syrups (containing e.g. about 10-50% sugar), and elixirs agent (containing about 20-50% ethanol), or parenteral administration is in the form of sterile injectable solutions or suspensions containing about 0.05-5% suspending agent in an isotonic medium. For example, these pharmaceutical preparations may contain from about 0.01% to 99%, more preferably from about 0.1% to 90% by weight of the active ingredient in admixture with the carrier.

The two active ingredients or pharmaceutical compositions of the present invention may be administered by conventional routes including, but not limited to: intramuscular, intraperitoneal, intravenous, subcutaneous, intracutaneous, oral, intratumorally or topical administration. Preferred routes of administration include oral, intramuscular or intravenous administration.

From the standpoint of ease of administration, preferred pharmaceutical compositions are liquid compositions, especially injections.

In addition, the two active ingredients or drugs of the present invention can also be used in combination with other drugs for preventing and/or treating liver immune disorders.

### The main advantages of the present invention include:

(1) The present invention has discovered for the first time that CST1 protein gene, or its protein, or its promoter or IFN-γ or its receptor inhibitor can significantly prevent and/or treat liver immune disorders.
(2) The present invention has discovered for the first time that the CST1 protein gene, or its protein, or its promoter or IFN-γ or its receptor inhibitor can also (a) improve the survival rate of mammals; and/or (b) significantly reduce serum alanine aminotransferase, aspartate aminotransferase, and/or blood ammonia levels; and/or (c) protect function of hepatic parenchymatous cells; and/or (d) inhibit the pro-inflammatory activation of IFN-γ on macrophages; and/or (e) inhibit the expression of a large number of ISGs downstream of IFN-γ; and /or (f) attenuate the efficiency of IFN-γ binding to IFNGR2
(3) The present invention has discovered for the first time that overexpression of CST1 can make ineffective cells obtain a certain therapeutic effect, which shows that CST1 protein itself and cell therapy based on CST1 overexpression have great potential for the treatment of acute liver failure.
(4) The present invention proves for the first time the therapeutic effect of CST1 protein on acute liver failure, and CST1 protein is an easy-to-produce protein product with wide potential uses.

The present invention will be further described below in conjunction with specific implementation. It should be understood that these examples are only used to illustrate the present invention and not to limit the scope of the present invention. The experimental method of unreceipted specific conditions in the following examples, usually according to normal conditions, people such as Sambrook, molecular cloning: conditions described in laboratory manual (New York: Cold Spring Harbor Laboratory Press, 1989), or according to manufacture conditions recommended by the manufacturer. Percentages and parts are by weight unless otherwise indicated.

The materials and reagents used in the examples are all commercially available products unless otherwise specified.

Human endodermal stem cells are obtained from a cell line independently established in our laboratory, and the method for establishing the cell line has been published (Cheng, X., Ying, L., Lu, L., Galvao, A.M., Mills, J.A., Lin, H.C., Kotton , D.N., Shen, S.S., Nostro, M.C., Choi, J.K., et al. (2012). Self-renewing endodermal progenitor lines generated from human pluripotent stem cells. Cell Stem Cell 10, 371-384.).

### General method

### (1) In vitro maintenance and expansion of human endodermal stem cells (hEnSCs)

The in vitro maintenance of hEnSCs can be carried out by culturing and passage in conditioned medium plated with Matrigel and a certain amount of irradiated Mouse Embryonic Fibroblast (MEF) (3×10⁵/10cm-dish) matrix. Quantitative expansion is performed. The conditioned medium consists of serum-free medium (Serum Free Medium, SFD) as the basal medium, with the addition of a certain amount of cytokines: bFGF (FGF2) (10ng/ml), EGF (10ng/ml), VEGF (10ng/ml) ml) and BMP4 (10 ng/ml) and the small molecule compound Kartogenin (1.5 µM/ml). 1L SFD consists of 250ml Ham's F12, 750ml Homemade IMDM, 5ml 10% BSA, 5ml N2 supplement, 10ml B27 supplement. The medium is changed every 2 days, and the passage is carried out every 5 days. The passage is generally carried out at a ratio of 1:4 or 1:5. The cells need to be digested for 5 minutes and then pipetted into single cells and mixed with MEF and then plated on a new culture dish. Plate at 1.5-2 × 10⁶/10cm-dish.

### (2) In vitro maintenance and expansion of human pluripotent stem cells/embryonic stem cells (Human Pluripotent Stem Cells/Human Embryonic Stem Cells; hPSCs/hES)

The strain of hES is H9, which is a stable human embryonic stem cell line established in the laboratory, referring to the following literature for the establishment method (Cheng, X., Ying, L., Lu, L., Galvao, A.M., Mills, J.A., Lin, H.C., Kotton, D.N., Shen, S.S., Nostro, M.C., Choi, J.K., et al. (2012). Self-renewing endodermal progenitor lines generated from human pluripotent stem cells. Cell Stem Cell 10, 371-384.). To culture hES, a layer of Matrigel diluted 1:6 with culture medium needs to be pre-coated in tissue culture dishes and pre-added 8 × 10⁵/10cm-dish irradiated mouse embryonic fibroblasts (Mouse Embryonic Fibroblast, MEF), and then seeded into cells. The cell culture medium is composed of basal medium DMEM/F12 50:50 (Invitrogen), 15 % KOSR (current lot: 771421), 1x NEAA, 1x P/S, 1x L-Glu, 1x Sodium Bicarbonate (8ml from stock), 1x Sodium Pyruvate (2.5 ml from stock), 1:550 dilution 2ME (55mM stock). The medium was changed daily until the cell density reached ~90% for passage. Aspirate the medium first, add TRYPLE Express w/Phenol Red to digest for 2 min, remove the TRYPLE, add hES medium, and gently scrape the cells with Soft Cell Scrapers and transfer into new tissue culture dishes at a ratio of 1:3-1:6.

### (3) CST1 gene lentiviral plasmid cloning and lentiviral packaging, infecting hES and screening hES overexpression of CST1.

Cystatin SN (CST1) protein is highly expressed in the definitive endoderm stage of human embryonic development, so that we used cDNA made from definitive endoderm induced by hES in vitro differentiation, cloned the CST1 gene from the cDNA, and ligated it into a lentiviral vector pWPI.1. Then we used the 293FT cell line as the cell line for packaging CST1 overexpression virus, and the pWPI.1-CST1 plasmid and the packaging plasmids pspAX2 and pMD2.G were transfected into 293FT in proportion by calcium transfection method, and after waiting for 2 days, collect the packaged virus in the supernatant. Add hES culture system to infect for 2 days (1:1 viral supernatant: hES medium). After 4 days of hES infection, the hES cells of pWPI.1-CST1 were infected by adding puromycin at a concentration of 1ug/ml to the hES medium until no large number of cells died when the medium was changed, and the remaining cells are the CST1-overexpressing hES lines, referred to as CST1⁺ES.

### (4) Methods of transplanting hEnSCs into animals

The experiment used WISTAR strain wild-type male rats (Rat) as the experimental subjects, all of which are injected with a single-cell suspension (PBS is a solution) of hEnSC (or hES, or CST1⁺ ES) with good viability through the hepatic portal vein (Portal Vein), the injection quantity is 1 × 10⁷/Rat. Before transplantation, the animal needs to be anesthetized with gas and maintained during the operation. During the operation, the abdominal cavity of the animal is opened with surgical instruments, the opening is as small as possible, the intestine on the hepatic portal vein is opened, and using insulin needle to inject single cell suspension along the hepatic portal vein into the liver, immediately after the needle is drawn, use a cotton swab to press the needle position to stop bleeding. After stopping the bleeding, return the intestines and suture the abdominal muscles and skin of the animal. Animals are observed after recovery and kept in a sterile environment to prevent infection after surgery.

### (5) Induction method of acute liver failure in animals

In the experiment, the compound D-galactosamine hydrochloride (D-GalN) was injected intraperitoneally to induce acute liver failure. The animals were fasted for one day before the injection, and the cells were transplanted 24 hours after the injection. The injection dose of rats was 1.4g/kg. After injection, rats had a high mortality rate within one week, and the peak death period was on the second and third days.

### (6) Method for detecting the efficacy of hEnSCs in the treatment of acute liver failure

It is mainly divided into two indicators: (a) Changes in the survival rate of animals, which are judged by observing the changes in the survival curve of multiple rats drawn within one week. (b) Detection of changes in animal serum liver function indexes, blood samples were collected from the heart, and blood samples were collected from rats within one week to detect serum AST, ALT, blood ammonia (Blood Ammonia), and prothrombin time (Prothrombin Time). The differences in liver function between treated and untreated groups were compared.

### (7) Rat macrophage isolation method:

The separation method is based on Percoll^{™} density gradient centrifugation. We put D-GalN-induced male rats with acute liver failure, two days after D-GalN administration, anesthetized them in a sterile environment, and opened the abdominal cavity to perfuse the liver. The perfusion needle was inserted through the portal vein of the rat, and the opening of the inferior vena cava was used as the outlet of the perfusate, and the peristaltic pump was used to continuously and stably perfuse the fluid. First perfuse 100ml of IX Hank's Balanced Salt Solution (HBSS) for about 10 minutes to flush out the blood in the liver. The rat liver was then cut out, the superficial blood was washed with IX HBSS, and the liver was minced with scissors. For one rat, minced liver was added to 20 ml RPMI-1640 medium containing 0.1% Collagenase IV + 0.01% DNaseI + 5% Fetal Bovine Serum (FBS) + 1% Penicillin-Streptomycin (P/S), digested with slow shaking in a 37°C water bath for one hour. After digestion, undigested tissue was filtered out with a 100 µm sieve. Centrifuged at 300g for 10min, resuspended with 20ml of 25% percoll solution, slowly adding it to a centrifuge tube containing 20ml of 50% percoll, then putting it in a centrifuge, centrifuged at 4°C, rotating speed at 1260g, centrifugation time for 15min, and adjusting the speed up and down to the lowest gear 0. After centrifugation, carefully removing a layer of cells between 25% percoll and 50% percoll, then adding RPMI medium, then centrifuged to wash off the residual Percoll solution, and resuspended with RPMI+10% FBS+1% P/S, incubated at 2.5 × 10⁵/well in 6-well-dish at 37°C for half an hour, and the non-adherent cells were washed away and replaced with a new medium (same as before), and the resulting adherent cells were macrophages.

### (8) Macrophage culture method:

The resulting primary macrophages were isolated and experiments were started immediately. Recombinant protein IFN-γ (IFNG) was added to the culture system at a final concentration of 100ng/ml, CST1 was added to the culture system at a final concentration of 500ng/ml, the medium was RPMI-1640+10% FBS+1% P/S, after 3 days of culture, removing the medium, and the adherent cells were lysed with RNA extraction lysate, RNA was extracted, and the expression of ISGs in macrophages under different conditions was detected.

### (9) Co-immunoprecipitation:

Co-immunoprecipitation was performed using the Pierce^{™} Classic Magnetic IP/Co-IP kit from Thermo Fisher, and the same amount of macrophage protein lysate (~1000ug) was used for each experiment. The products obtained by immunoprecipitation were detected by western blot and antibodies against IFN-γ (IFNG) and IFNGR2, and the grayscale analysis was performed by ImageJ software.

### Example 1 Analysis of therapeutic efficacy and active ingredients of human endodermal stem cells (hEnSCs) for acute liver failure

In order to find an alternative therapy for liver transplantation for the treatment of acute liver failure, and cell therapy is one of the potential therapies, so after trying a variety of potential cell types, we have found a new stem cell line with good therapeutic effect on acute liver failure, namely human endodermal stem cells (hEnSCs). Through animal experiments in rats with acute liver failure, we have found that transplantation of hEnSCs through the hepatic portal vein can significantly improve the survival rate of animals within one week, and the effect is slightly better than that of mesenchymal stem cells (MSCs) known to have a certain efficacy in acute liver failure (Fig. 1A) and the liver function indexes of alanine aminotransferase (ALT), aspartate aminotransferase (AST) and blood ammonia (Blood Ammonia) are significantly decreased, indicating that transplantation of hEnSCs plays a good protective effect on the liver function of rats with acute liver failure. (Figure 1B).

Since hEnSCs transplantation has a good effect on acute liver failure in animal experiments, in order to facilitate the treatment of acute liver failure, the molecular mechanism behind the treatment of acute liver failure by hEnSCs is more worthy of study.

In order to understand the unique molecular mechanism of hEnSCs in the treatment of acute liver failure, the present invention attempts to search for genes that are specifically highly expressed only in hEnSCs, so as to screen possible key therapeutic factors. By comparing the single-cell transcriptome sequencing data of hEnSCs, namely endodermal stem cells, with their upstream and downstream cells in the differentiation pathway: human embryonic stem cells (ES), human hepatoblasts (Hepatoblast), and human hepatocytes (Hepatocyte), some hEnSCs-specific and highly expressed genes are identified (Fig. 1C). Among these genes, the objects to be screened are genes with immunomodulatory or anti-apoptotic effects and positive effects on liver function protection. Through extensive screening, the CST1 gene is screened, the full name of which is Cystatin SN, is a secreted protein. It is preliminarily speculated that CST1 may play an immunoregulatory role in the treatment of hEnSCs.

### Example 2 Efficacy verification experiment of CST1 protein on acute liver failure

In order to verify what we thought might be a key molecule for the treatment of acute liver failure by hEnSCs, we decided to overexpress the CST1 gene in a cell line that does not express and has no therapeutic effect on acute liver failure. If the cell line can obtain a certain therapeutic effect on acute liver failure, it means that the CST1 gene has a certain therapeutic effect on acute liver failure.

The cell line is human embryonic stem cells (ES), and then the CST1 overexpression plasmid vector is packaged by lentivirus, and ES is infected to establish a CST1 overexpression ES cell line, namely CST1⁺ ES. Then we examined whether the CST1 gene was overexpressed in the established cell lines, and compared its expression in hEnSCs, ES, and MSCs. As shown in Figure 2A, CST1⁺ES overexpressed the CST1 gene, but the expression level was still not as high as that in hEnSCs, while ES and MSCs did not express CST1.

We then used CST1⁺ ES to treat acute liver failure in rats and compared it with a cell line known to be effective, hEnSCs, Hepatocyte, and ES, a cell line known to be ineffective. Comparisons were still made in terms of survival and liver function indicators. Among the 5 rats with acute liver failure transplanted with CST1⁺ ES, 3 survived within one week, while all 5 rats with common ES died (Fig. 2B). Compared with hEnSCs and Hepatocyte, it still has better therapeutic effect, but CST1⁺ ES does have a significant improvement in therapeutic effect compared with common ES. In terms of liver function indicators, although CST1⁺ ES is inferior to hEnSCs and Hepatocyte, it has lower serum ALT and AST than common ES, indicating that CST1⁺ ES also has a certain protective effect on liver function. In view of the fact that the expression of CST1 in CST1⁺ES is still lower than that of hEnSCs, a good therapeutic effect has been obtained, which also reflects the importance of CST1 for the therapeutic efficacy of hEnSCs. Compared with common ES, CST1⁺ ES also has a significant reduction in alanine aminotransferase (ALT) and aspartate aminotransferase (AST) indicators, which means that CST1⁺ ES has a certain degree of liver function protection due to the expression of CST1. effect (Figure 2C).

In conclusion, it can be confirmed that CST1 protein has a good therapeutic effect on acute liver failure in rats, and the therapeutic effect of CST1 protein on acute liver failure has never been reported. Overexpression of CST1 alone can make ineffective cells obtain a certain therapeutic effect, which shows that CST1 protein itself and cell therapy based on CST1 overexpression have great potential for the treatment of acute liver failure.

### Example 3 Study on the mechanism of action of CST1

The present invention not only finds out that CST1 in transplanted animals has the effect of immunoregulating and increasing the survival rate of animals with acute liver failure, but also explores the mechanism of CST1 immunoregulatory in the liver of animals, and explains some mechanisms of CST1 immune regulation in liver.

It is known that macrophages in the liver are abundant in the liver and play an important role in the pathogenesis of acute liver failure. Macrophages have a strong pro-inflammatory effect in the process of acute liver failure, and will recruit other immune cells such as monocytes, neutrophils, helper T cells, etc., increasing the burden on the liver and causing a more severe immune response, thereby aggravating acute liver failure. Interferon-y (IFNγ) is one of the main pro-inflammatory activators of macrophages. The downstream genes (Interferon stimulated genes, ISGs) activated by interferon-y and other types of interferon signaling are collectively referred to as ISGs. ISGs are highly activated in hepatic macrophages in rats with acute liver failure.

As shown in Figure 3A, macrophages in the liver of rats with acute liver failure were extracted, cultured in vitro, and the addition of IFN-γ signal can significantly increase the RNA level expression of ISGs, simulating the immune state of macrophages in vivo. However, when 500ng/ml of CST1 recombinant protein was added into the macrophage culture system for 3 days, the activation effect of IFN-γ on ISGs in macrophages was greatly weakened. Among the highly expressed ISGs listed in the figure, Ifit2, Ifit3, Mx2, Irf7, Eif2ak2, Ifitm3, Isg15 and Sp100 are all significantly decreased. This experiment proves from the cellular level that CST1 can inhibit the pro-inflammatory activation of IFN-γ on macrophages, as it can inhibit the expression of a large number of ISGs downstream of IFN-γ.

Not only that, the role of CST1 was also studied at the molecular level. Based on the fact that CST1 can inhibit the expression of ISGs downstream of IFN-γ signaling, we suspect that CST1 may be able to affect or block a certain link in the IFN-γ signaling pathway. Therefore, we studied the receptor of IFN-γ gene, which has two different subunits, IFNGR1 and IFNGR2. As shown in the left panel of Figure 3B, Co-Immunoprecipitation is used to find the binding of IFN-γ to one of its receptor subunits, IFNGR2. After CST1 recombinant protein is added to the system, the binding efficiency becomes lower by about 50%, as shown in the right panel of Figure 3B. Specifically, the IFN-γ (IFNG) antibody is used to co-precipitate the protein that can bind to IFN-γ in the protein lysate of rat macrophages. IFNGR2, as the protein receptor of IFN-γ, naturally binds to IFN-γ and co-precipitated, and detected by western blotting; but if CST1 recombinant protein is added to rat macrophage proteolysis (5ug is added to 1000ug protein lysate), after co-precipitation of IFN-γ and IFNGR2, the ratio of IFNGR2 protein to IFN-γ protein detected by western blotting, as shown in the right panel of Figure 3B, which indicates that CST1 protein has an interfering effect on the binding between IFN-γ and IFNGR2, and CST1 inhibits the pro-inflammatory activation of macrophages by reducing the binding efficiency of IFN-γ and IFNGR2, thereby reducing the expression of ISGs downstream of IFN-γ, thereby achieving its immunoregulatory effect.

### Discussion

Finding an alternative to liver transplantation has always been a hot spot in the field of acute liver failure research, and cell therapy is currently a very promising treatment strategy. However, regardless of the treatment strategy, the treatment of acute liver failure relies on the study of the molecular mechanism behind the therapeutic efficacy. Without the exact molecular mechanism and effective therapeutic factors, the application and optimization of the therapy cannot be carried out.

The inventors have not only discovered the therapeutic effect of hEnSCs cell level on acute liver failure, but also further discovered that CST1 is a key factor in the treatment of acute liver failure by hEnSCs.

CST1 is a little-studied secreted protein. In the known literature, CST1 is a cysteine protease inhibitor, abundant in saliva and tears, but not expressed in most human tissues. CST1 has certain effects on anti-allergy and antibacterial, but its efficacy on acute liver failure has never been reported. Therefore, the present invention discovers the therapeutic effect of CST1 on acute liver failure, and invents a treatment method for acute liver failure based on CST1.

Since CST1 is not expressed in most human tissues, cells known to have therapeutic effects on acute liver failure, such as human mesenchymal stem cells (MSCs), have different therapeutic molecular mechanisms. This also shows that CST1 treatment of acute liver failure is a novel and unique therapy.

Overexpression of the CST1 gene alone can make ES cells without therapeutic efficacy obtain a good therapeutic effect, then CST1 may be combined with some cells that have therapeutic efficacy themselves, such as MSCs, to obtain better therapeutic efficacy.

All publications mentioned herein are incorporated in the present application by reference as if each document is individually cited as a reference. It should also be understood that, after reading the above teachings of the present invention, those skilled in the art can make various changes or modifications, equivalents of which fall in the scope of claims as defined in the appended claims.

## Claims

1. Use of a *CST1* gene, or a protein or a promoter thereof for preparing a composition or preparation for preventing and/or treating liver immune disorders.

2. The use of claim 1, wherein the composition or preparation is also used for one or more purposes selected from the group consisting of:
(a) increasing the survival rate of a mammal;
(b) significantly lowering levels of alanine aminotransferase, aspartate aminotransferase, and/or blood ammonia in serum;
(c) protecting the function of hepatic parenchymatous cell;
(d) inhibiting the pro-inflammatory activation of IFN-γ on a macrophage;
(e) inhibiting the expression of a large number of ISGs downstream of IFN-γ;
(f) attenuating the efficiency of IFN-γ binding to IFNGR2.

3. The use of claim 1, wherein the CST1 promoter is selected from the group consisting of a small molecule compound, a vector expressing CST1, and a combination thereof.

4. The use of claim 1, wherein the *CST1* gene or a protein thereof is derived from an endodermal stem cell or definitive endoderm.

5. The use of claim 1, wherein the CST1 protein is selected from the group consisting of:
(A) a polypeptide whose amino acid sequence is as shown in SEQ ID NO.: 1;
(B) a CST1 protein derivative or an active fragment thereof formed by the amino acid sequence as shown in SEQ ID NO.: 1 through the substitution, deletion or addition of one or several (usually 1-60, preferably 1-30, more preferably 1-20, most preferably 1-10) amino acid residues;
(C) compared with the amino acid sequence as shown in SEQ ID NO.: 1, a CST1 protein derivative or an active fragment thereof with homology ≥90%, preferably ≥ 95%, more preferably ≥98%, most preferably ≥99%.

6. Use of an inhibitor of IFN-γ or a receptor thereof for preparing a composition or preparation for preventing and/or treating liver immune disorders.

7. A cell preparation, comprising:
(a1) a first pharmaceutical composition containing (a) a first active ingredient, the first active ingredient being the CST1 gene, or a protein or a promoter thereof; or an inhibitor of IFN-γ or a receptor thereof, and a pharmaceutically acceptable carrier;
(a2) an optional second pharmaceutical composition containing (b) a second active ingredient, the second active ingredient being a stem cell that does not express the CST1 gene or a protein thereof; and a pharmaceutically acceptable accepted carrier.

8. The cell preparation of claim 7, wherein in the cell preparation, the concentration of the component (a1) is 100-1000ug/ml, preferably 200-1000ug/ml, more preferably 300-1000ug/ml.

9. The cell preparation of claim 7, wherein in the cell preparation, the concentration of the component (a2) is 1.0×10⁶-1.0×10⁸ cells/ml, preferably 5.0×10⁶-1.0×10⁸ cells/ml, more preferably 1.0×10⁷-1.0×10⁸ cells/ml.

10. A kit, comprising:
(i) a first container, and the active ingredient (a1) the CST1 gene, or a protein thereof, or a promoter thereof; or an inhibitor of IFN-γ or a receptor thereof, located in the first container, or a medicament containing the active ingredient (a); and
(ii) an optional second container, and the active ingredient (a2) a stem cell that does not express the CST1 gene or a protein thereof located in the second container, or a medicament containing active ingredient (a2).

11. A method for screening a potential therapeutic agent for preventing and/or treating liver immune disorders, comprising:
(a) in a test group, in a culture system, in the presence of a test compound, culturing a cell expressing a *CST1* gene for a period of time T1, and detecting the expression level E1 of the *CST1* gene in the culture system of the test group;
and in a control group without the test compound and other conditions being the same, detecting the expression level E2 of the CST1 gene in the culture system of the control group; and
(b) comparing E1 and E2, if E1 is significantly higher than E2, indicating that the test compound is a potential therapeutic agent for liver immune disorders.

12. A method for screening a potential therapeutic agent for preventing and/or treating liver immune disorders, comprising:
(a) in a test group, in a culture system, in the presence of a test compound, culturing a cell for a period of time T1, and detecting the binding of the IFN-γ to its receptors in the culture system of the test group;
and in a control group without the test compound and with the same other conditions, detecting the binding of the IFN-γ to its receptor in the culture system of the control group;
(b) if the binding efficiency E1 of the IFN-γ to its receptor in the test group is significantly lower than the binding efficiency E2 of the IFN-γ to its receptor in the control group, indicating that the test compound is a candidate compound.
